# FASCICULE DE BREVET EUROPEEN

(11) **EP 0 888 379 B1**
(45) Date de publication et mention de la délivrance du brevet: **10.01.2001**
(21) Numéro de dépôt: 97907154.5
(22) Date de dépôt: 28.02.1997
(51) Int. Cl.: C07J 41/00, A61K 31/575, A61K 9/127, C12N 15/88

(54) **COMPOSES APPARENTES A LA FAMILLE DES AMIDINIUMS, COMPOSITIONS PHARMACEUTIQUES LES CONTENANT ET LEURS APPLICATIONS**
GUANIDINIUM-VERBINDUNGEN, PHARMAZEUTISCHE PRÄPARATE DAVON UND IHRE VERWENDUNGEN
COMPOUNDS RELATED TO THE AMIDINIUM FAMILY, PHARMACEUTICAL COMPOSITIONS CONTAINING SAME, AND USES THEREOF

(30) Priorité: 01.03.1996 FR 9602604; 30.07.1996 FR 9609557
(43) Date de publication de la demande: 07.01.1999
(73) Titulaire: CENTRE NATIONAL DE LA RECHERCHE SCIENTIFIQUE (CNRS), 75016 Paris (FR)
(72) Inventeur: LEHN, Jean-Marie, F-67000 Strasbourg (FR); LEHN, Pierre, F-75019 Paris (FR); VIGNERON, Jean-Pierre, F-91790 Boissy-sur-Saint-Yon (FR)
(74) Mandataire: Savina, Jacques
(86) Numéro de dépôt international: FR9700364
(87) Numéro de publication internationale: WO9731935

(56) Documents cités:
- WO-A-93/05162
- WO-A-96/01840
- WO-A-96/18372
- JOURNAL OF BIOLOGICAL CHEMISTRY, vol. 270, no. 52, 29 Décembre 1995, MD US, pages 31391-31396, XP002029823 J. K. GUY-CAFFEY ET AL: "Novel Polyaminolipids Enhance the Cellular Uptake of Oligonucleotides"
- NUCLEOSIDES & NUCLEOTIDES, vol. 14, no. 3-5, 1995, pages 951-955, XP002029824 K. JAYARAMAN ET AL: "Approaches to Enhance the Binding Affinity and Nuclease Stability of Triplex Forming Oligonucleotides"
- CHEMICAL ABSTRACTS, vol. 122, no. 11, 13 Mars 1995 Columbus, Ohio, US; abstract no. 123040, GAGNE ET AL: "Liposome Mediated Gene Delivery. III. In Vitro Toxicity of Cationic Liposome DNA Plasmid Complexes" page 89; colonne 1; XP002029825 & PROC. INT. SYMP. CONTROLLED RELEASE BIOACT. MATTER, 21ST, 1994, pages 236-237,
- PROCEEDINGS OF THE NATIONAL ACADEMY OF SCIENCES OF USA, vol. 93, no. 18, 3 Septembre 1996, WASHINGTON US, pages 9682-9686, XP002030510 J. P. VIGNERON ET AL: "Guanidinium-Cholesterol Cationic Lipids: Efficient Vectors for the Transfection of Eukaryotic Cells"
- C. DENSMORE ET AL: "", JOURNAL OF GENE MEDECINE PREPRINT, , 1999, Vol. 1, no. 4, pages 1 à 20

## Description

La présente invention concerne de nouveaux composés apparentés à la famille des amidiniums dont en particulier les guanidiniums, des compositions pharmaceutiques les contenant et leurs applications.

Plus précisément, la présente invention se rapporte à des composés de formule générale I et leurs sels 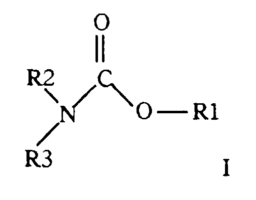 dans laquelle:
- R1 représente un dérivé du cholestérol,
R2 et R3 représentent indépendamment l'un de l'autre un atome d'hydrogène ou un groupement de formule générale II avec au moins l'un d'entre eux différent d'un atome d'hydrogène, 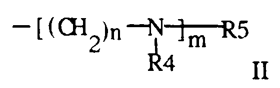 dans laquelle:
- n et m représentent indépendamment l'un de l'autre et de manière distincte entre les groupements R2 et R3 un entier compris entre 0 et 4,
- R4 et R5 représentent indépendamment l'un de l'autre un atome d'hydrogène ou un groupement de formule III, avec au moins l'un d'entre eux différent d'un atome d'hydrogène, 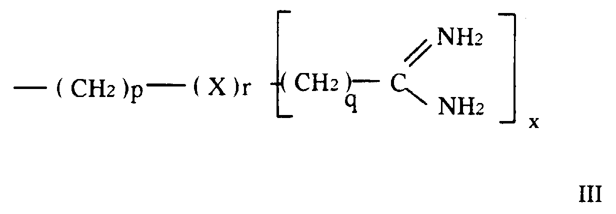
dans laquelle
p et q représentent, indépendamment l'un de l'autre, un entier compris entre 0 et 4 et r est égal à 0 ou 1, avec pour r égal à 1
X représentant un groupement NH et x étant alors égal à 1 ou
X représentant un atome d'azote et x étant alors égal à 2 et
avec p, q et r pouvant varier indépendamment entre les groupements R4 et R5.

A titre de sous-famille préférée, on citera plus particulièrement dans le cadre de la présente invention les composés de formule générale I dans laquelle R2 ou R3 représentent, indépendamment l'un de l'autre, un atome d'hydrogène ou un groupement de formule IV 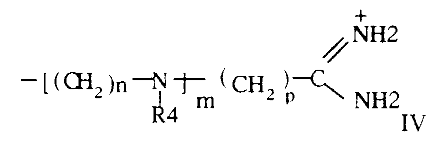 dans laquelle
n, m et p sont définis comme précédemment et R4 représente un atome d'hydrogène ou un groupement de formule V 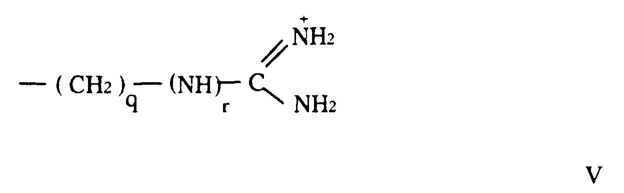
avec q et r définis comme précédemment,
et avec m, n, p, q et r pouvant varier de manière indépendante entre les différents groupements R2 et R3.

Ces nouveaux produits de formule générale (I) peuvent se présenter sous forme de sels non toxiques et pharmaceutiquement acceptables. Ces sels non toxiques comprennent les sels avec les acides minéraux (acides chlorhydrique, sulfurique, bromhydrique, phosphorique, nitrique) ou avec les acides organiques (acides acétique, propionique, succinique, maléique, hydroxymaléique, benzoïque, fumarique, méthanesulfonique ou oxalique) ou avec les bases minérales (soude, potasse, lithine, chaux) ou organiques (amines tertiaires comme la triéthylamine, la pipéridine, la benzylamine).

A titre représentatif des composés selon l'invention on peut plus particulièrement citer les composés de sous-formules générales suivantes:

(H₂N)₂⁺C-NH-(CH₂)₂-NH-COO-R1 VI

[(H₂N)₂⁺C-NH-(CH₂)₃][(H₂N)₂C⁺-NH-(CH₂)₄]-N-COO-R1 VII

[(H₂N)₂⁺C-NH-(CH₂)₂]₂-N-(CH₂)₂-NH-COO-R1 VIII

[(H₂N)₂⁺C-(CH₂)₂]₂N-COO-R1 IX

[[(H₂N)₂⁺C-(CH₂)₂]₂-N-(CH₂)₂]₂N-COO-R1 X

dans lesquelles R1 possède la définition précédente.

A titre représentatif des guanidiniums et amidiniums revendiqués, on peut plus particulièrement citer les composés des sous formules précédentes VII ,VIII et IX avec R1 y représentant un groupement cholestéryle. Les trois composés seront respectivement identifiés ci-après sous la désignation BGSC ( Bis Guanidino Spermidine Cholestérol ) et BGTC ( Bis Guanidino Tren Cholestérol) et BADC (bis chlorhydrate de Bis Amidinium Diéthylènetriamine Cholesterol ).

Les composés revendiqués sont tout particulièrement intéressants sur le plan thérapeutique pour leurs caractères non toxiques et leur propriétés amphiphiles. Compte -tenu de ces qualités, il peuvent notamment être utilisés pour la complexation d'acides nucléiques dans la perspective d'une transfection cellulaire de ceux-ci. Ces composés peuvent donc être avantageusement employés en thérapie génique.

Le J. Biol. Chem. Vol 270(52) p. 31391-6 (1995) décrit des agents de transfection du type polyamine-cholesterol, sans groupements amidinium.

Les composés VII (BGSC) et VIII (BGTC) sont tout particulièrement avantageux pour le transfert de gène in vivo. Ces deux composés se complexent avec l'ADN et le protègent contre la dégradation due aux variations de pH lors du transport vers la cellule à traiter.

L'invention concerne donc une composition pharmaceutique qui comprend au moins un composé selon l'invention. Dans un mode de réalisation préféré de l'invention le composé est le Bis Guanidino Spermidine Cholesterol (BGSC) et dans un autre mode préféré le composé est le Bis Guanidino Tren Cholesterol (BGTC).

La thérapie génique a pour principal objectif de corriger des maladies génétiques associées à un défaut d'expression et/ou une expression anormale, c'est à dire déficiente ou excessive, d'un ou plusieurs acides nucléiques. On tente de suppléer à ce type d'anomalies génétiques par le biais de l'expression cellulaire in vivo ou in vitro de gènes clonés.

Aujourd'hui, plusieurs méthodes sont proposées pour la délivrance intracellulaire de ce type d'information génétique. L'une des technologies actuellement mises en oeuvre, repose précisément sur l'emploi de vecteurs chimiques ou biochimiques. Ces vecteurs synthétiques ont deux fonctions principales, compacter l'ADN à transfecter et promouvoir sa fixation cellulaire ainsi que son passage à travers la membrane plasmique et, le cas échéant, à travers les deux membranes nucléaires. Des lipides cationiques chargés positivement comme le chlorure de N-[1-(2,3-dioleyloxy)propyl]-N,N,N-triméthylammonium (DOTMA) ont ainsi été proposés. Avantageusement, ils interagissent, sous la forme de liposomes ou de petites vésicules, spontanément avec de l'ADN, qui est lui chargé négativement, pour former des complexes lipides-ADN, capables de fusionner avec les membranes cellulaires, et permettent ainsi la délivrance intracellulaire de l'ADN. Toutefois, dans le cas particulier du DOTMA, sa bonne efficacité au niveau de la transfection demeure malheureusement associée à un défaut de biodégradabilité et un caractère toxique à l'égard des cellules.

Depuis le DOTMA, d'autres lipides cationiques ont été développés sur ce modèle de structure : groupe lipophile associé à un groupement amino via un bras dit "spacer". Parmi ceux-ci, on peut plus particulièrement citer ceux comprenant à titre de groupement lipophile deux acides gras ou un dérivé du cholestérol, et comportant, en outre, le cas échéant, à titre de groupement amino, un groupement d'ammonium quaternaire. Les DOTAP, DOBT ou le ChOTB peuvent notamment être cités à titre représentatifs de cette catégorie de lipides cationiques.

De part leur structure chimique et leur caractère biodégradable, les composés revendiqués répondent précisément aux exigences requises pour un vecteur de transfection d'acides nucléiques.

La présente invention vise donc également toute application de ces nouveaux composés dans la transfection in vitro, ex vivo et/ou in vivo de cellules et notamment pour la vectorisation d'acides nucléiques. Elle se rapporte en particulier à toute composition pharmaceutique comprenant, outre au moins un composé selon l'invention, un acide nucléique.

Dans les compositions de la présente invention, l'acide nucléique associé à au moins un composé revendiqué peut être aussi bien un acide désoxyribonucléique qu'un acide ribonucléique. Il peut s'agir de séquences oligonucléotidiques ou polynucléotidiques d'origine naturelle ou artificielle, et notamment d'ADN génomique, d'ADNc, d'ARNm, d'ARNt, d'ARNr, de séquences hybrides ou de séquences synthétiques ou semi-synthétiques. Ces acides nucléiques peuvent être d'origine humaine, animale, végétale, bactérienne, virale, etc. Ils comportent de préférence un gène thérapeutique.

Un autre objet de l'invention porte donc sur une composition pharmaceutique contenant en outre un acide nucléique. Préférentiellement cet acide nucléique est soit un acide désoxyribonucléique soit un acide ribonucléique. Dans un mode préféré de réalisation de l'invention l'acide nucléique comporte un gène thérapeutique.

Au sens de l'invention, on entend par gène thérapeutique notamment tout gène codant pour un produit protéique ayant un effet thérapeutique. Le produit protéique ainsi codé peut être une protéine, un peptide, etc. Ce produit protéique peut être homologue vis-à-vis de la cellule cible (c'est-à-dire un produit qui est normalement exprimé dans la cellule cible lorsque celle-ci ne présente aucune pathologie). Dans ce cas, l'expression d'une protéine permet par exemple de pallier une expression insuffisante dans la cellule ou l'expression d'une protéine inactive ou faiblement active en raison d'une modification, ou encore de surexprimer ladite protéine. Le gène thérapeutique peut aussi coder pour un mutant d'une protéine cellulaire, ayant une stabilité accrue, une activité modifiée, etc. Le produit protéique peut également être hétérologue vis-à-vis de la cellule cible. Dans ce cas, une protéine exprimée peut par exemple compléter ou apporter une activité déficiente dans la cellule, lui permettant de lutter contre une pathologie, ou stimuler une réponse immunitaire.

Parmi les produits thérapeutiques au sens de la présente invention, on peut citer plus particulièrement les enzymes, les dérivés sanguins, les hormones, les lymphokines : interleukines, interférons, TNF, etc (FR 9203120), les facteurs de croissance, les neurotransmetteurs ou leurs précurseurs ou enzymes de synthèse, les facteurs trophiques : BDNF, CNTF, NGF, IGF, GMF, aFGF, bFGF, NT3, NT5, HARP/pléiotrophine, etc la dystrophine ou une minidystrophine (FR 9111947), la protéine CFTR associée à la mucoviscidose, les gènes suppresseurs de tumeurs : p53, Rb, Rap1A, DCC, k-rev, etc (FR 93 04745), les gènes codant pour des facteurs impliqués dans la coagulation : Facteurs VII, VIII, IX, les gènes intervenant dans la réparation de l'ADN, les gènes suicides (thymidine kinase, cytosine déaminase), les gènes de l'hémoglobine ou d'autres transporteurs protéiques, les gènes correspondant aux protéines impliquées dans le métabolisme des lipides, de type apolipoprotéine choisie parmi les apolipoprotéines A-I, A-II, A-IV, B, C-I, C-II, C-III, D, E, F, G, H, J et apo(a), les enzymes du métabolisme comme par exemple la lipoprotéine lipase, la lipase hépatique, la lécithine cholestérol acyltransférase, la 7 alpha cholestérol hydroxylase, la phosphatidique acide phosphatase, ou encore des protéines de transfert de lipides comme la protéine de transfert des esters de cholesterol et la protéine de transfert des phospholipides, une protéine de liaisons des HDL ou encore un récepteur choisi par exemple parmi les récepteurs LDL, récepteurs des chylomicrons-remnants et les récepteurs scavenger.etc.

L'acide nucléique thérapeutique peut également être un gène ou une séquence antisens, dont l'expression dans la cellule cible permet de contrôler l'expression de gènes ou la transcription d'ARNm cellulaires. De telles séquences peuvent, par exemple, être transcrites dans la cellule cible en ARN complémentaires d'ARNm cellulaires et bloquer ainsi leur traduction en protéine, selon la technique décrite dans le brevet EP 140 308. Les gènes thérapeutiques comprenent également les séquences codant pour des ribozymes, qui sont capables de détruire sélectivement des ARN cibles (EP 321 201).

Comme indiqué plus haut, l'acide nucléique peut également comporter un ou plusieurs gènes codant pour un peptide antigénique, capable de générer chez l'homme ou l'animal une réponse immunitaire. Dans ce mode particulier de mise en oeuvre, l'invention permet donc la réalisation soit de vaccins soit de traitements immunothérapeutiques appliqués à l'homme ou à l'animal, notamment contre des microorganismes, des virus ou des cancers. Il peut s'agir notamment de peptides antigéniques spécifiques du virus d'Epstein Barr, du virus HIV, du virus de l'hépatite B (EP 185 573), du virus de la pseudo-rage, du "syncitia forming virus, d'autres virus ou encore spécifiques de tumeurs (EP 259 212).

Préférentiellement, l'acide nucléique comprend également des séquences permettant l'expression du gène thérapeutique et/ou du gène codant pour le peptide antigénique dans la cellule ou l'organe désiré. Il peut s'agir des séquences qui sont naturellement responsables de l'expression du gène considéré lorsque ces séquences sont susceptibles de fonctionner dans la cellule infectée. Il peut également s'agir de séquences d'origine différente (responsables de l'expression d'autres protéines, ou même synthétiques). Notamment, il peut s'agir de séquences promotrices de gènes eucaryotes ou viraux. Par exemple, il peut s'agir de séquences promotrices issues du génome de la cellule que l'on désire infecter. De même, il peut s'agir de séquences promotrices issues du génome d'un virus. A cet égard, on peut citer par exemple les promoteurs des gènes E1A, MLP, CMV, RSV, etc. En outre, ces séquences d'expression peuvent être modifiées par addition de séquences d'activation, de régulation, etc. Il peut aussi s'agir de promoteur, inductible ou répressible.

Par ailleurs, l'acide nucléique peut également comporter, en particulier en amont du gène thérapeutique, une séquence signal dirigeant le produit thérapeutique synthétisé dans les voies de sécrétion de la cellule cible. Cette séquence signal peut être la séquence signal naturelle du produit thérapeutique, mais il peut également s'agir de toute autre séquence signal fonctionnelle, ou d'une séquence signal artificielle. L'acide nucléique peut également comporter une séquence signal dirigeant le produit thérapeutique synthétisé vers un compartiment particulier de la cellule.

Les lipides cationiques décrits dans la présente invention, et, en particulier, le BGTC et le BGSC, sont capables de se complexer avec l'ADN, et représentent une alternative avantageuse aux vecteurs viraux pour le transfert d'acides nucléiques d'intérêt thérapeutique, in vitro, ex vivo ou in vivo. Du fait des propriétés chimiques des groupes guanidinium, et en particulier de leur pKa élevé, ces lipides cationiques sont capables de protéger les molécules d'ADN contre les dégradations dues aux variations de pH. Les résultats présentés dans les exemples montrent que ces composés permettent la transfection de nombreux types cellulaires, avec une grande efficacité. L'efficacité de la transfection dépend en particulier du rapport lipide cationique / ADN dans les complexes formés. Un rapport entre les deux composés particulièrement favorable pour la transfection consiste à avoir 6 à 8 groupes guanidinium pour un groupe phosphate sur l'ADN.

L'efficacité de transfection des complexes lipide cationique /DNA peut en outre être améliorée par addition de lipide neutre, et formation de liposomes cationiques. Ces liposomes sont formés par un complexe entre le lipide cationique et le lipide neutre. Celui-ci peut être choisi parmi :
- la dioléoylphosphatidyléthanolamine (DOPE),
- l'oléoyl-palmitoylphosphatidyléthanolamine (POPE),
- le di-stéaroyl, -palmitoyl, -mirystoyl phosphatidyléthanolamine
- leurs dérivés N-méthylés 1 à 3 fois;
- les phosphatidylglycérols,
- les diacylglycérols,
- les glycosyldiacylglycérols,
- les cérébrosides (tels que notamment les galactocérébrosides),
- les sphingolipides (tels que notamment les sphingomyélines)
- et les asialogangliosides (tels que notamment les asialoGM1 et GM2).

On utilise préférentiellement la DOPE.

Préférentiellement, le composé de l'invention et le lipide neutre sont présents dans un rapport compris entre 1 et 5, plus préférentiellement 2 et 4. En outre le rapport lipide total (composé de l'invention plus lipide neutre) sur DNA est avantageusement choisi de sorte que le rapport net de charges positives soit compris entre 2 et 5. Particulièrement avantageusement, le rapport est de 3 environ.

L'invention concerne donc également toute composition pharmaceutique comprenant un composé selon l'invention, un lipide neutre et un acide nucléique. Préférentiellement le composé selon l'invention est choisi parmi le BGTC et le BGSC. Tout aussi préférentiellement le lipide neutre est la DOPE. L'acide nucléique est soit un acide désoxyribonucléique soit un acide ribonucléique. Préférentiellement l'acide nucléique comporte un gène thérapeutique.

Outre cette application des dérivés d'amidinium revendiqués, il est également possible d'envisager leur valorisation dans les applications suivantes: interférence au niveau d'interactions entre acides nucléiques et protéines résultant dans une inhibition ou stimulation de certains processus par exemple de régulation d'expression génétique ou d'activité enzymatique ( polymérase, transcriptase, etc....), complexation sélective d'espèces anioniques pour leur extraction, leur élimination ou leur détection à l'aide de sondes/électrodes à membrane par exemple, utilisation comme réactif de laboratoire pour effectuer des opérations de transfection in vitro, etc.

En conséquence, la présente invention a également pour objet toute application thérapeutique des dérivés d'amidinium tels que décrits précédemment, soit directement, soit au sein de compositions pharmaceutiques.

De préférence, les compositions pharmaceutiques de l'invention contiennent également un véhicule pharmaceutiquement acceptable pour une formulation injectable, notamment pour une injection directe au niveau de l'organe désiré, ou pour une administration par voie topique (sur peau et/ou muqueuse). Il peut s'agir en particulier de solutions stériles, isotoniques, ou de compositions sèches, notamment lyophilisées, qui, par addition selon le cas d'eau stérilisée ou de sérum physiologique, permettent la constitution de solutés injectables.

La présente invention sera plus complètement décrite à l'aide des exemples et figures qui suivent, qui doivent être considérés comme illustratifs et non limitatifs.

### FIGURES

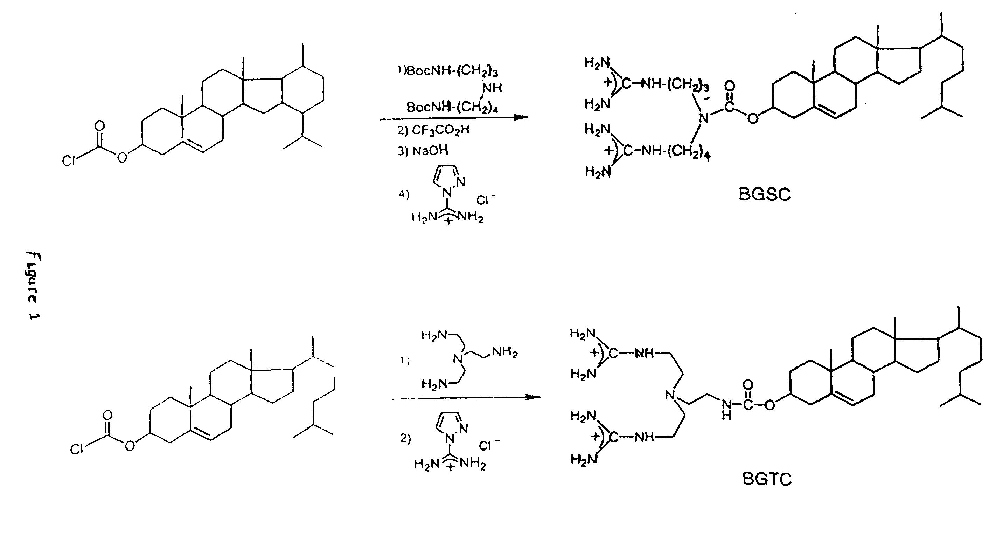

Figure 1 : Représentation schématique des protocoles opératoires de préparation du BGSC et BGTC.

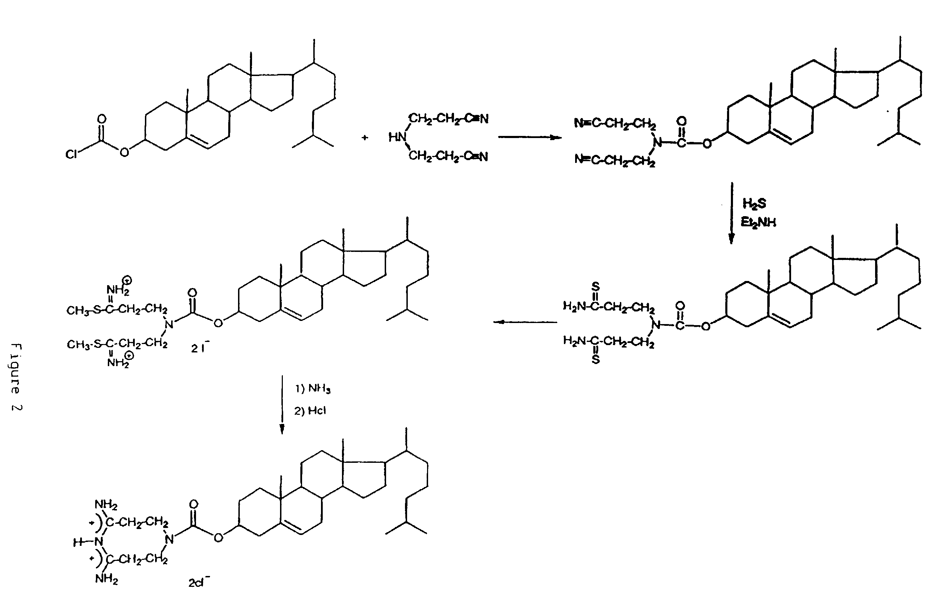

Figure 2 : Représentation schématique des protocoles opératoires de préparation du BADC.

Figure 3 : Mesure de l'efficacité de transfection en fonction du rapport composé lipidique/ADN.

Figure 4 : Mesure de l'efficacité de transfection en fonction du rapport lipides totaux / ADN.

Figure 5 : Mesure de l'effet sérum sur l'efficacité de transfection de cellules HepG2 (5A), HeLa (5B), NIH 3T3 (5C) et 293 (5D). Barres grises : Présence de sérum; Barres vides : transfections en absence de sérum pendant 2h.

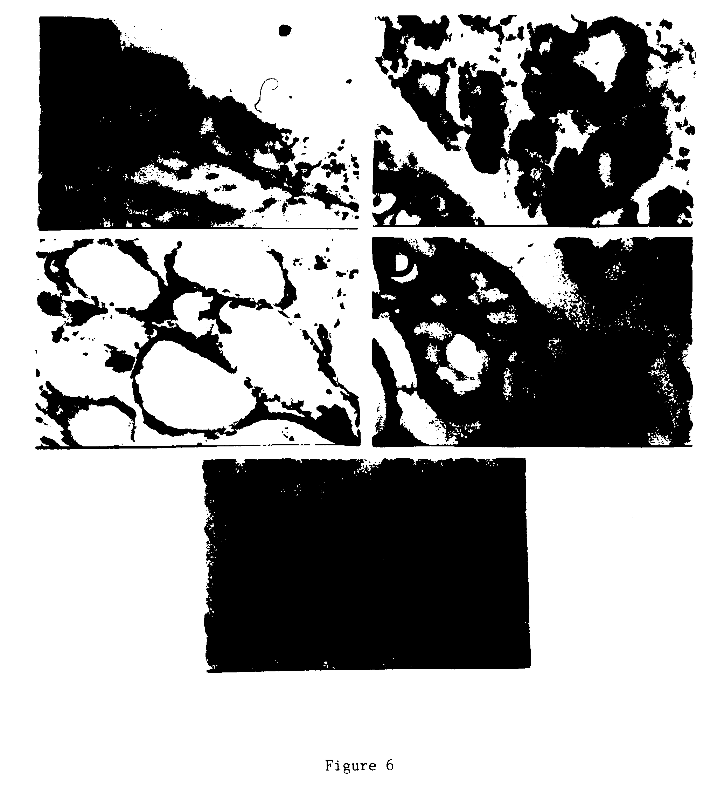

Figure 6 : Photographies de cryosections de trachées murines montrant l'expression de gène reporter dans l'épithélium de l'appareil respiratoire après transfection in vivo avec des liposomes BGTC/DOPE. (6A) et (6B) : Détection de l'expression de la beta-galactosidase dans des cellules transfectées, localisées à la surface de l'épithelium (6A) et dans les glandes sous mucosales (6B); (6C) : Détection des cellules exprimant la beta-galactosidase dans les glandes sous-mucosales par marquage à l'immunoperoxidase en utilisant un anticorps monoclonal anti-beta-galactosidase; (6D) : détection des cellules exprimant la luciférase dans les glandes sous-mucosales par marquage à l'immunoperoxidase en utilisant un anticorps polyclonal anti-luciférase; (6E) : coloration à l'immunoperoxidase: contrôle négatif effectué en absence d'anticorps.

Figure 7 : Mesure de l'efficacité de transfert de gènes in vivo par injection intraveineuse d'un complexe ADN/BGTC-liposome.

Figure 8 : Mesure de l'efficacité de transfert de gènes in vivo par injection intrapéritonéale d'un complexe ADN/BGTC-liposome.

### A- PREPARATION DE DERIVES SELON L'INVENTION

### MATERIEL ET METHODES

### 1. Mesures physiques

Les spectres de résonance magnétique nucléaire du proton ( RMN 1H ) ont été enregistrés sur un spectromètre Bruker AC200. Les déplacements chimiques sont exprimés en ppm par rapport au TMS.

### 2. Chromatographies sur silice

.Les chromatographies sur couche mince (CCM) ont été effectuées sur des plaques de gel de silice Merck de 0,2 mm d'épaisseur

.Les chromatographies sur colonne ont été effectuées sur gel de silice 60 Merck de granulométrie 0,040-0,063mm.

### EXEMPLE 1:

### PREPARATION DU GUANIDINO SPERMIDINE CHOLESTEROL BGSC

### -1; Préparation du Di-Boc carbamate (1)

On ajoute une solution de chloroformate de cholesteryle(0,9g, 2mmol) et de Et₃N (0,214g, 2mmol) dans CH₂Cl₂ (40ml) à une solution de ¹N,⁸N-Boc₂-spermidine (0,690g, 2mmol) dans CH₂Cl₂ (20ml). Après reflux pendant 5 heures, le mélange est lavé à l'eau (2x50ml), séché sur Na₂SO₄ et le solvant est évaporé. Le produit brut est purifié par chromatographie sur un gel de silice utilisant CH₂Cl₂/MeOH 95/5 comme éluant pour donner du pur carbamate (1,3g, 86%).

¹H NMR, δ(CDCl₃, 200 Mhz) ; 0,5-2,5(m, structure de cholesteryl, Boc signal et groupes centraux CH2 depuis le spermidine), 3,1-3,3(m, 8H, N-CH2), 4.50(m,1H,H_{3α}), 5,37(d,1H,H₆).

### - 2. Préparation de l'aminocarbamate(2)

On dissout le composé (1) (1,3g) dans 20ml de CH₂Cl₂ et l'on ajoute à cette solution refroidie (lit de glace) 2ml de CF₃CO₂H fraîchement distillé pour libérer les groupes protecteurs BOC. Après agitation à température ambiante pendant 3 heures, on ajoute 50ml de ¹N NaOH et le mélange est extrait avec CH₂Cl₂ Les couches organiques sont lavées à l'eau, séchées sur Na₂SO₄ et évaporées pour obtenir l'aminocarbamate (2) brut (0,945g, 98%).

¹H NMR, δ(CDCl₃, 200 Mhz) ; 0,5-2,5(m, structure de cholesteryl et groupes centraux depuis le spermidine), 2,7(m, 4H, CH₂-N-C0-), 3,25 (large m,4H,N-CH₂), 4,49(m,1H,H_{3α}), 5,35(d,1H,H₆).

### 3.- Synthèse du BGSC.

On dissout le carbamate (2) brut (0,94g) dans 30ml de THF/MeOH 85/15. Puis on ajoute du 1H-pyrazolecarboxamidine (0,495g, 3,4mmol) et du diisopropylethylamine (0,436, 3,4mmol) dans 30ml de THF/MeOH 85/15. Après agitation à température ambiante pendant 18 heures on ajoute lentement 250 ml de diethylether et on sépare le précipité obtenu par décantation. Le composé brut est mis en suspension trois fois dans du diethylether et séparé par décantation pour donner du GSC pur (0,570g, 47%).

1H NMR δ(DMSO d6) 200 MHz ; 0,5-2,5(m, structure du cholesteryl et CH₂ central), 3,1(m, 8H, N-CH₂), 4,3(m,1H,H_{3α}), 5,3(d,1H,H₆) ; 7,2 (large s, 8H,NH₂+), 7,8(s,2H,NH), Anal. Calc. pour C₃₇H₆₇N₇O₂,2HCl,C,62,15 ; H,9,67 ; N,13,71.

Résultat d'analyse élémentaire: C,62,28 ; H,9,81 ; N,13,15.

### EXEMPLE 2:

### PREPARATION DU GUANIDINO TREN CHOLESTEROL, BGTC

### 1.préparation de l'Aminocarbamate (5)

On ajoute lentement une solution de chloroformate de cholesteryle (1,8g, 4mmol) dans 100 ml de CH₂Cl₂ à une saturation de tris(2-aminoethyl)amine(TREN) (11,68 g, 80 mmol) dans 400 ml de CH₂Cl₂. Après agitation à température ambiante pendant deux heures, on libère l'amine inerte par lavage à l'eau (3x100ml) et après séchage sur Na₂SO₄ on évapore le solvant. On isole (5) sous forme de trihydrochloride de la manière suivante : on dissout le produit brut dans 10ml de MeOH et l'on ajoute goutte à goutte une solution saturée de HCl methanolique (5ml) ; le précipité obtenu est séparé par décantation et, pour obtenir un composé pur, est mis en suspension trois fois dans MeOH et séparé par décantation. Après séchage sous vide le trihydrochloride (6) pur est obtenu (1,71g, 64%).

1H NMR δ(CDCl₃ + εCD₃OD, 200MHz). 0,5-2,4(m, structure de cholesteryl), 2,5(m,2H,CH₂NHCO), 2,8(large s,4H,+HN-CH₂), 3,06(large s,4H,CH₂NH₃+), 3,20(m,2H,⁺HN-CH₂), 4,4(m,1H,H_{3α}), 5,3(d,1H,H₆), Anal. Calc. pour C₃₄H₆₂N₄O₂,3HCl ;C,61,10 ; H,9,80 ; N,8,38. Résultat : C,61,25 ; H,9,96 ; N,8,22.

### 2- Préparation du B.GTC

On ajoute lentement une solution de chloroformate de cholesteryle (2,24g, 5mmol) dans 100ml de CH₂Cl₂ à une large saturation de tris(2-aminoéthyl)amine(TREN)(29,2g, 200mmol) dans 250ml de CH₂Cl₂. Après agitation à température ambiante pendant 2 heures, on libère l'amine inerte par lavage à l'eau (3x100ml) et après séchage sur Na₂SO₄, le solvant est évaporé. Le produit brut (3,2g) est dissous dans THF/MeOH 50/50 (20ml) ; puis l'on ajoute au mélange du 1H-pyrazole-1-carboximidine (1,465g,10mmol) et du diisopropylamine (1,3g, 10mmol). Après agitation à température ambiante pendant 18 heures, on ajoute du diethylether et le précipité obtenu est séparé par décantation. Afin d'obtenir un échantillon pur, le composé brut est mis en suspension trois fois dans du diethylether et séparé par décantation (2,15g), 60% après séchage sous vide.

1H NMR(DMSOd6, 200MHz) ; 0,5-2(m, structure du cholesteryl), 2,2(m,2H,N-CH₂), 2,6(large s,4H,N-CH₂), 3.0(d,2H,N-CH₂), 3,2(large s,4H), 4,3(m,1H,H_{3α}), 5,3(d,1H,H₆), 7,3(large s,8H,NH₂+), (7,8(large s,2H,NH). Anal. Calc. pour C₃₆H₆₆N₈O₂,2HCl ; C,60,39 ; H,9,57 ; N,15,65. Résultat : C,60,38 ; H,9,67 ; N,15,56.

### EXEMPLE 3:

### PREPARATION DU BIS-CHLORHYDRATE DE BIS AMIDINIUM BACD.

### 1.Préparation du dinitrile

On ajoute une solution de chloroformate de cholestéryle (8,97g; 20mmol.) dans du CH₂Cl₂ (100ml) goutte à goutte à une solution d'iminopropionitrile (2,46g, 20mmol.) et Et₃N ( 2.02 g, 20 mmol dans du CH₂Cl₂ (100ml). Après agitation à la température ambiante, pendant 6 heures, on lave à l'eau (2x 100ml) et sèche sur Na₂SO₄. Le solvant est évaporé et le produit brut (10,5g) est recristallisé dans le méthanol. (8,59g; 80%).

1H NMR δ(CDCl₃, 200MHz). 0,5-2,5(m, structure de cholesteryl), 2,7 (m, 4H; -CH₂CN), 3,62 (m, 4H, -CH2-N-), 4,50 (m,1H,H_{3α} ), 5,39(d,1H,H₆).

### 2. Préparation du dithioamide

Dans une solution du dinitrile, préparé comme décrit précédemment, ( 0,324g, 6mmol.) et de diéthylamine ( 0,884g; 12mmol) dans du DMF (30ml), maintenue à 50°C, on fait barboter pendant 2 heures un léger courant de H₂S. On verse la solution bleue sur de la glace et on sépare le précipité ainsi obtenu par filtration. On dissout ce produit brut dans du CH₂Cl₂ et lave à l'eau (2x50ml). Après séchage sur Na₂SO₄, le solvant est évaporé et le produit obtenu recristallisé deux fois dans du méthanol (1,86g; 51,5%).

1H NMR(DMSOd6, 200MHz) ; 0,5-2,5(m, structure du cholesteryl), 2,67(t,4H,N-CH₂), 3,50(t,4H,S=C-CH₂), 4,3(m,1H,H_{3α})5,33(d,1H,H₆).

### 3. Préparation du bis-iodure de dithioamidate

A une solution de dithioamide (0,603g; 1mmol) dans de l'acétone (20ml), on ajoute de l'iodure de méthyle (2ml) et on laisse reposer à la température ambiante pendant 48 heures. On filtre le précipité obtenu (0,741g; 83,5%). Ce produit est utilisé directement sans purification annexe.

### 4. Préparation du bis chlorhydrate de bis amidinium BADC.

Dans une suspension au reflux du bis-iodure (0,741g; 0,83mmol.), tel qu'obtenu précédemment, dans de l'isopropanol (10ml) on fait barbotter un courant de NH₃ pendant 2 heures puis on continue de chauffer au reflux 4 heures supplémentaires. On évapore l'isopropanol et le résidus est repris dans du méthanol (10ml). Après passage de cette solution sur une résine échangeuse d'ions basique, on fait passer un courant d'acide chlorhydrique dans l'éluant et on évapore à sec. Le bis chlorhydrate ainsi obtenu, est dissous dans le minimum d'éthanol ( environ 5ml). On filtre un léger insoluble et on rajoute au filtrat un large excès d'éther diéthylique. On obtient une poudre que l'on filtre et l'on sèche (320mg; 60%). Une recristallisation dans l'isopropanol permet d'isoler le produit attendu, sous une forme analytiquement pure.

1H NMR(DMSOd6, 200MHz) ; 0,5-2,6(m, structure du cholesteryl et-CH₂-amidinium), 3,57 (s, large 4H, N-CH₂) 4,3(m,1H,H_{3α}),5,3(d,1H,H₆), 8,64 (s, large; 2H; NH₂), 9,07 (s; large; 1H; NH), 9,18 (s; large; 2H).

Anal. Cal. Pour C₃₄H₅₆N₄O₂, 2HCl: C, 65,25. H, 9,32; N, 8,94. Résultat C, 65,40. H, 9,87; N, 9,55.

### B: UTILISATION DE DERIVES SELON L'INVENTION POUR LE TRANSFERT DE GENES IN VITRO ET IN VIVO

### MATERIELS ET METHODES

1- Les essais de transfection qui suivent sont effectués avec du BGSC et du BGTC préparés selon les protocoles décrits dans les exemples 1 et 2.

### 2 - Préparation des liposomes

Un mélange de lipide cationique et de DOPE (dans un rapport molaire de 3/2) dans du chloroforme (CHCl₃) est évaporé sous vide et remis en suspension dans une solution de tampon HEPES 20 mM à PH 7,4, sous une atmosphère N2.

La concentration finale en lipide est de 1,2 mg/ml.

Le mélange est vortexé pendant 5 mn, puis « soniqué » pendant 5 mn avec un sonificateur (Branson Ultrasonic 2210), enfin stocké a + 4°C pendant 24 h pour hydratation.

La dispersion résultante est « soniquée » de nouveau (Sonifier Branson 450) pendant 5 à 10 mm pour former des liposomes.

Après centrifugation, la solution est filtrée sur un filtre de diamètre de pore 0.22µ (Millex GS, Millepore) et stockée à + 4°C.

La taille des liposomes contenant BGSC ou BGTC a été étudiée à l'aide d'un appareil à diffraction laser (Autosizer 4700, Malvern Instruments). Cette étude montre un pic unique correspondant à un diamètre moyen de 50nm en analyse multimodale par nombres.

### 3 - Cultures cellulaires

L'origine (espèce et tissus) de la plupart des lignées cellulaires utilisées pour les expériences de transfection est donnée dans le tableau I.

Les cellules de la lignée cellulaire HeLa (P Briand, ICGM Paris) sont dérivées d'un carcinome humain d'origine épitheliale cervicale.

Les cellules NIH 3T3 (C Lagrou, Institut Pasteur, Lille) sont des fibroblastes de souris.

La lignée cellulaire NB2A (C. Gouget, Paris) est derivée d'un neuroblastome de souris.

Toutes les cellules à l'exception des cellules AtT-20 et PC12, ont été cultivées dans du milieu Dulbecco modifié (DMEM, GIBCO) complementé avec 10 % de sérum de veau fetal (FCS; GIBCO), de la pénicilline à 100 unités/ml (GIBCO) et de la streptomycine (GIBCO) à 100µg/ml.

Les cellules AtT-20 ont été cultivées dans du DMEM/F12 (GIBCO) complementé avec 10 % de sérum de veau fetal.

Les cellules PC12 ont été cultivées dans du DMEM complementé avec 10 % FCS et 5 % de sérum de cheval.

Toutes les cellules ont été maintenues en culture dans des boîtes plastiques (Falcon) sous atmosphère humide à 5 % de CO2.

### 4 - Plasmides

Les plasmides pRSV-Luc (O. BENSAUDE, ENS, Paris), et pRSV-nlsLacZ ont été amplifiés dans E. Coli et préparés par purification sur gradient CsCl par des techniques standard. Dans le plasmide pRSV-nlsLacZ, et le plasmide pRSV-Luc, le gène LacZ d'E.coli et sa séquence signal de localisation nucléaire et le gène luciférase sont respectivement sous le contrôle transcriptionnel du LTR/RSV (Rous Sarcoma Virus). Le plasmide pXL2774 comporte le gène codant pour la luciférase sous contrôle du promoteur du cytomégalovirus (CMV).

### 5 - Protocole de transfection in vitro

Le jour précédent la transfection 2 X 10⁵ cellules sont déposées par puits (plaques à 6 puits Falcon).

Chaque puits contient une lignée cellulaire différente.

Ainsi au jour de la transfection les cellules sont a demi confluentes.

L'ADN plasmidique (5µg) et la quantité désirée de lipide bis-guanidinium ont été chacun dilués dans 250µl de DMEM sans FCS et vortexé. Après environ 5 min les deux solutions ont été laissées à incuber à température ambiante pendant 15min. Le mélange de transfection est alors ajouté aux cellules (0,5 ml par puits) qui ont été lavées avec un milieu sans sérum. Après 4 à 6 heures d'incubation à 37°C, 1 ml de milieu avec sérum est ajouté dans chaque puits sans que l'on enlève le mélange de transfection. 24 heures après la transfection, le milieu est remplacé par 1 ml de milieu de culture frais. Les cellules sont recueillies 2 jours après la transfection pour mesurer l'expression de la luciferase.

Des transfections témoins ont été faites en utilisant des agents de transfections commercialisés :
- la lipopolyamine Transfectam ® (JP Behr, Strasbourg, France) a été utilisée en solution alcoolique à un rapport optimum de charges ioniques Transfectam ®/DNA 6-8.
- la Lipofectin ® (Life Technologies Inc., Cergy Pontoise, France) qui est une formulation de liposomes ayant pour lipide neutre la DOPE et pour lipide cationique le DOTMA.(N[1(2,3-dioleyloxy) propyl]-N, N, N- trimethylammonium chloride).

Les complexes ADN/Liposomes ont été obtenus dans les conditions standard recommandées par le fabricant.
- des cellules ont été transfectées par précipitation au phosphate de calcium. (Keown, W.A., Campbell, C.R. ; Kucherlapati, R.S. (1990) *Methods in Enzymology ;* Academic Press : New York, **185**, 527-537).

### 6 - Mesure de la luciferase pour les transfections réalisées in vitro

L'activité luciferase est mesurée 48 heures après transfection en utilisant une variante la procédure de De Wet et al. (De Wet, J.R., Wood, K.V., DeLuca, M., Helinski, D.R. & Subramani, S. (1987) *Mol. Cell. Biol.* **7**, 725-737.)
- on retire le milieu de culture
- les cellules sont lavées avec une solution saline de tampon phosphate froid
- puis elles sont lysées par incubation dans 250 µl de tampon de lyse (25mM triphosphate pH7;8, 8 mM MgCl₂, 1mM dithiotreitol, 15 % de glycerol et 1 % de triton X-100).
- on obtient un lysat clair après élimination des matériaux insolubles par centrifugation (15mm a + 4°C) dans une microcentrifugeuse.
- un Aliquot (20µl) d'extrait cellulaire est dilué dans 100µl de tampon de lyse auquel on ajoute 9µl d'ATP 25 mM (sigma) et 20µl de luciferine 25mM (sigma).
- les échantillons sont placés dans un luminomètre (Lurmet LB9501 Berthold, Nashua, NH) et on mesure l'émission lumineuse pendant 10 secondes.

L'étalonnage de la courbe RLU/Luciferase a été fait en utilisant differentes dilutions de luciferase de luciole purifiée (sigma) et montre que la partie linéaire de la courbe va de 10⁴ a 10⁷ RLU (relative light units).

La concentration en protéine a été mesurée par le test BCA (acide bicinchoninique) en utilisant la serumalbumine bovine comme témoin.

Les données pour l'activité luciférase sont exprimées en RLU/mg de protéine.

### 7 - Protocole d'injection in vivo au niveau des voies respiratoires de souris

Les souris utilisées sont des souris mâles OF1 (poids 30g) issues de Iffa-Credo (Lyon, France). Des liposomes cationiques BGTC/DOPE (rapport molaire 3:2) sont utilisés. Le mélange de transfection est obtenu à partir de 10µg d'ADN plasmidique (dans10µl eau), 20µl de liposomes cationiques dans un milieu Hepes 20mM (pH 7.4), à une concentration lipidique totale de 5mg/ml. Les agrégats ADN/lipides ainsi formés possèdent un rapport charge de l'ordre de 6.

Après anesthésie des souris avec du pentobarbital, le mélange de transfection (30µl) est injecté dans les voies respiratoires par instillation intratrachéeale via une canule insérée dans le lumen trachéal. 48h après l'instillation les animaux sont sacrifiés par overdose de pentobarbital et les poumons et trachées prélevés pour analyse.

### 8- Coloration à l'X-gal

Pour doser l'expression de la beta-galactosidase d'E.coli dans les cultures primaires, on imprègne les cellules pendant 15 minutes à 4°C avec une solution de paraformaldéhyde à 4% et on les incube ensuite avec de l'X-gal de substrat beta-galactosidase chromogénique (Sigma). La coloration bleue des noyaux des cellules transfectées est dosée par microscopie par luminescence.

### 9-Dosage des cellules X-gal au niveau des voies respiratoires transfectées in vivo

Pour la détection des cellules X-gal au niveau des voies respiratoires, les poumons et trachées sont traités 1h dans du paraformaldehyde à 4%, immergés ensuite toute une nuit dans du PBS contenant 30% de sucrose, puis incorporés dans un milieu de congélation et congelés à l'azote liquide. Des sections de cryostats de 5µ sont ensuite colorées pour l'activité de β-galactosidase par incubation toute une nuit avec du réactif X-gal (Sigma).

### 10- Dosage de la luciferase au niveau des voies respiratoires transfectées in vivo

Pour doser l'activité luciférase à l'issue des transfections in vivo, des fragments de tissus sont mis en présence de 30µl de tampon de lyse (25mM Tris-phosphate pH7.8, 1mM dithiothreitol (DTT), 15% glycerol et 1% Triton X-100), lysés en environ 1min avec un homogéniseur (Polylabo, Strasbourg, France) et conservés sur glace. Après centrifugation, 20µl de lysat est utilisé pour le dosage. L'activité luciferase est exprimée en RLU par mg de protéine.

### 11 - Protocole d'immunohistochimie

Des cryosections d'échantillons de poumons et trachées sont incubées 1h avec soit des anticorps murins monoclonaux anti- β-galactosidase d'E coli à 5µg/ml (Genzyme, Cambridge, MA) ou des anticorps de lapin polyclonaux 1:400 dilution (Promega).

La protéine β-galactosidase d'Escherichia coli est dosée par coloration à l'immunoperoxidase en utilisant un anticorps secondaire anti Ig de souris marqué à la biotine (Boehringer) et un conjugué streptavidin-POD (Boehringer). La protéine luciferase est dosée par coloration à l'immunoperoxidase en utilisant un anticorps de chèvre anti-lapin (ICN Biomedicals, Inc) et le système peroxidase-antiperoxidase de lapin (PAP) (Dako Glostrup, Denmark). Les sections sont examinées par microscopie luminescente. Des contrôles négatifs sont effectuées sans anticorps.

### EXEMPLE 4:

### TRANSFECTIONS IN VITRO A DES RAPPORTS AGENT DE TRANSFECTION/ADN DIFFERENTS

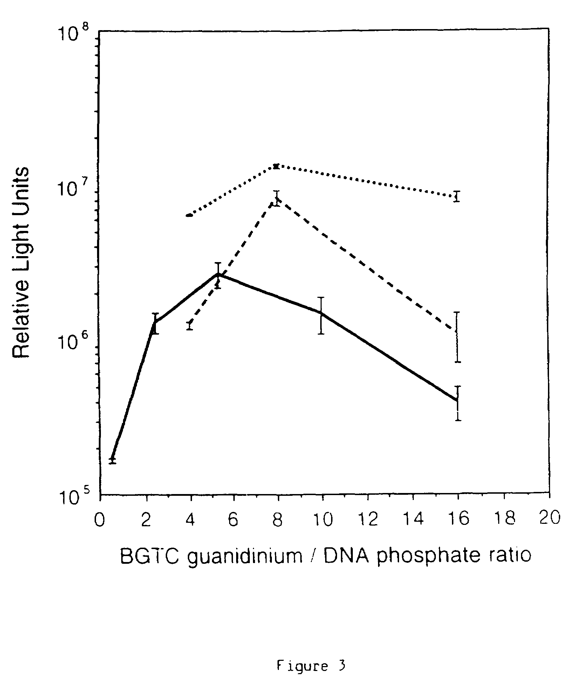

Afin d'optimiser l'efficacité de transfection obtenue avec des agrégats BGTC/ADN, la demanderesse a étudié l'influence du rapport BGTC/ADN sur le taux de transfection dans 3 types différents de cellules de mammifères connus pour leur relative sensibilité au méthodes de transfection classique. Les résultats sont présentés en figure 3.

Des expériences de transfection ont donc été réalisées avec des fibroblastes murins 3T3 des cellules épithéliales humaines HeLa et des neuroblastomes de souris NB2A. Au cours de ces expériences une certaine quantité fixe de plasmide pRSV-Luc a été mise en contact avec des quantités variables de BGTCen solution. Pour déterminer la fourchette de rapport à étudier la demanderesse est partie du principe qu'un µg d'ADN équivaut à 3 nm de phosphate chargé négativement et que seulement deux groupes guanidinium sont chargés positivement au pH neutre de formation des agrégats et de transfection. On mesure l'activité luciférase 48h après la transfection. L'expression de la luciférase est maximale pour des agrégats contenant de 6 (cellules HeLa) à 8 (cellules 3T3, NB2A) groupes guanidinium pour un groupe phosphate de l'ADN c'est à dire des agrégats ayant une charge positive importante.

### EXEMPLE 5

### TRANSFECTIONS IN VITRO AVEC DIFFERENTS TYPES CELLULAIRES.

Le transfert de gènes par l'intermédiaire de lipides cationiques est une technique de transfection attractive non seulement du fait qu'elle ne nécessite pas d'intermédiaires mais aussi parce qu'elle permet de transfecter une très grande variété de cellules de tissus et d'organismes différents. Pour étudier l'étendue de l'efficacité du BGTC comme agent de transfection plusieurs lignées cellulaires d'origines diverses ont été testées. Les résultats sont exposés dans le tableau 1 ci-après. Le rapport de charges a été choisi entre 6 et 8 pour une efficacité maximale. Afin d'établir une comparaison, des cellules des mêmes lignées ont également été transfectées en utilisant une lipopolyamine d'une part et d'autre part du phosphate de calcium. Les résultats montrent que le BGTC est normalement aussi efficace que le Transfectam et deux fois plus efficace que le phosphate de calcium.

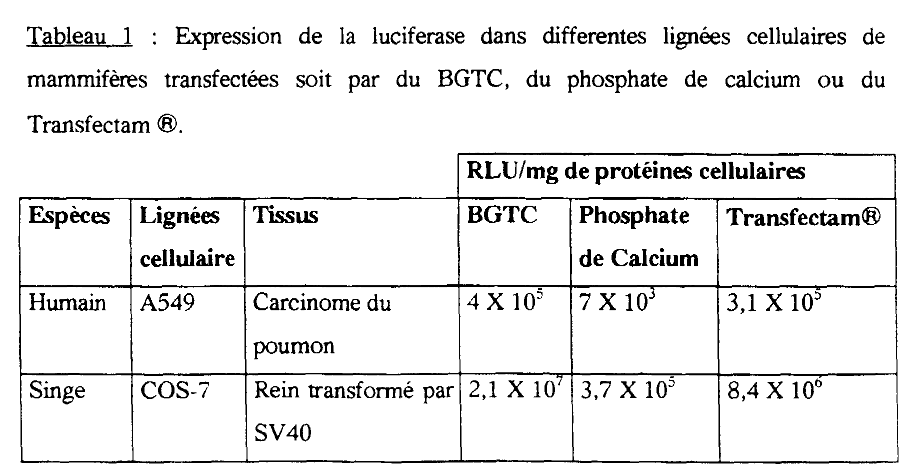

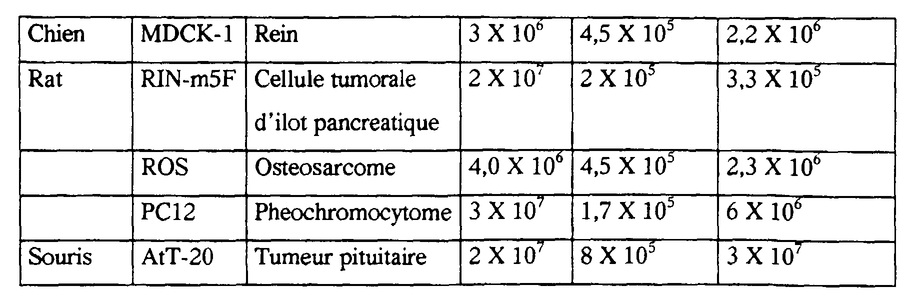

Toutes les transfections ont été faites au moins deux fois (n≥2).

### EXEMPLE 6

### TRANSFECTIONS IN VITRO EN PRESENCE D'UN LIPIDE NEUTRE

Le composé BGSC étant moins soluble en milieu aqueux que le BGTC la demanderesse a utilisé le premier sous forme de liposomes en présence d'un lipide neutre la DOPE (dioléoylphosphatidyléthanolamine). Les formulations sont préparées dans un rapport molaire 3/2. Une deuxième préparation contenant cette fois ci du BGTC a également été préparée avec de la DOPE dans les mêmes conditions.

### 6.1 - Détermination du rapport lipide total / ADN.

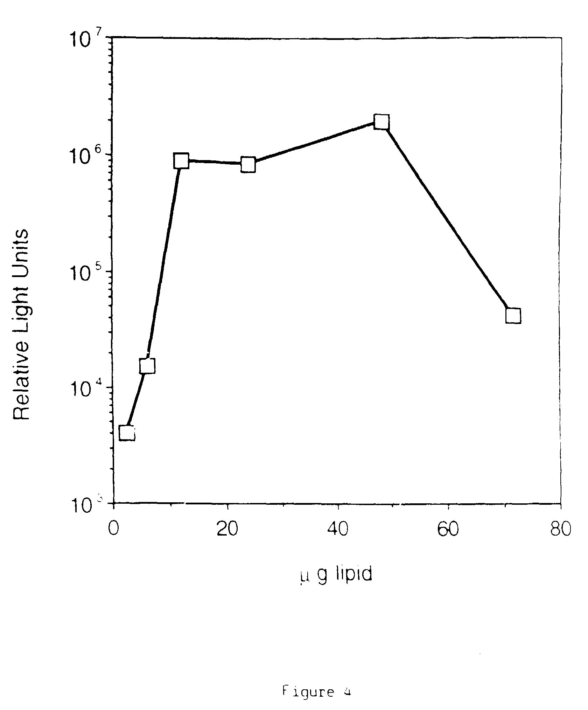

La demanderesse a tout d'abord déterminé le rapport lipide/ ADN au cours d'une expérience de transfection sur des cellules HeLa. La courbe obtenue est représentée figure 4. Les optima de transfection sont obtenus pour des rapports liposomes BGSC/ADN compris entre 1.5 et 5 avec un centre à 2.5-3. Les groupes guanidinium étant protonés à pH neutre, les meilleurs agrégats BGSC-DOPE/ADN ont une charge positive d'environ 3 (figure 4).

Les meilleurs rapports BGSC-DOPE/ADN sont donc inférieurs à ceux obtenus avec des agrégats de type BGTC/ADN (entre 6 et 8). La transfection en présence de liposomes cationiques est facilitée par la présence de DOPE et les propriétés fusogéniques de celle-ci.

### 6.2 - Transfection de différentes lignées cellulaires par des liposomes cationiques.

La demanderesse a procédé à des expériences de transfection dans différentes lignées cellulaires en utilisant des formulations de liposomes cationiques BGSC-DOPE et BGTC-DOPE dans des rapports molaires 3/2. Le rapport de charges guanidinium des lipides / phosphates de l'ADN est d'environ 2.5 (∼3) pour ces deux systèmes de transfert de gènes. Une transfection avec la Lipofectin® sert de témoin. Les résultats sont exposés dans le tableau 2 ci-dessous.

Ces résultats montrent que les dérivés du cholestérol portant des groupes guanidiniums sous forme de liposomes cationiques sont au moins aussi efficaces pour la transfection que la Lipofectin. Ces résultats peuvent bien entendu être optimisés pour chaque lignée cellulaire.

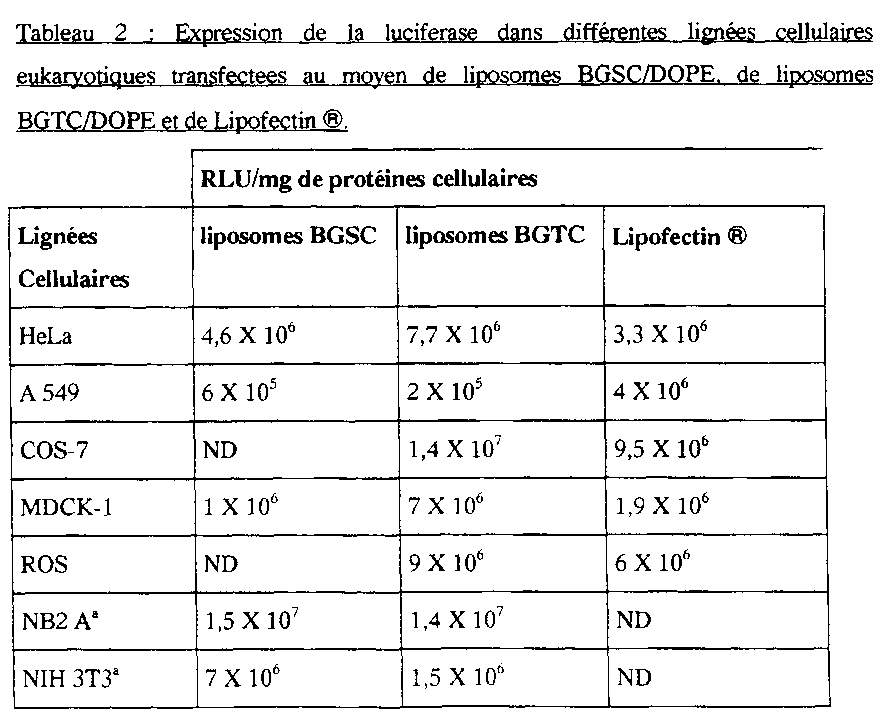

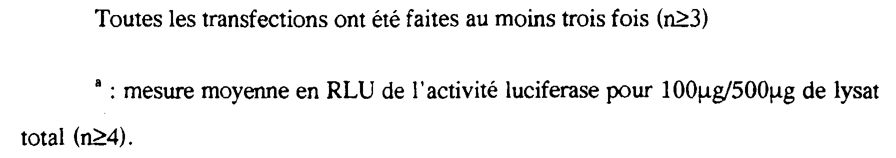

### EXEMPLE 7

### ETUDE DE L'EFFET DE LA PRESENCE DE PROTEINES SERIQUES SUR L'EFFICACITE DU TRANSFERT DE GENES IN VITRO

La plupart des compositions de transfection à base de lipides cationiques présentent une efficacité diminuée en présence de sérum. Cet exemple a pour objet de tester la sensibilité des composés de l'invention à l'effet sérum.

### 7.1 - Formulations des lipides cationiques

Les lipides décrits dans l'invention sont solubilisés dans de l'éthanol.
i) des solutions de « liposomes » sont obtenus en présence de DOPE comme suit : la DOPE (AVANTI) en solution dans le chloroforme est ajoutée aux solutions de lipide dans l'éthanol, dans un rapport molaire lipide cationique/DOPE de 3/2. Après évaporation à sec les mixtures sont reprises dans l'eau et chauffées à 50°C pendant 30 minutes ;
ii) des solutions de « micelles » sont obtenues en suivant le protocole décrit pour l'obtention de « liposomes » mais la solution de DOPE est remplacée par du chloroforme.

Les solutions sont toutes ajustées à 10 mM en lipide cationique.

### 7.2 - Acide Nucléique

L'acide nucléique utilisé est le plasmide pXL2774.

### 7.3 - Mélanges cytofectants (préparation extemporanée)

L'ADN est dilué à 20 µg/ml dans du NaCl 150 mM et les différentes formulations de lipide cationique sont diluées dans l'eau à 40, 80,120 et 160 µM. Les solutions d'ADN et de lipide sont mélangées volume à volume ce qui donne des ratios en nmoles/µg ADN respectivement de 2, 4, 6 et 8 ; la concentration saline est de 75 mM

### 7.4. - Transfections

Les cellules sont cultivées dans les conditions appropriées en microplaques de 24 puits (2 cm²/puits) et sont transfectées alors qu'elles sont en phase exponentielle de croissance et à 50-70 % de la confluence. Les cellules sont lavées par 2 fois 500 µl de milieu dépourvu de protéines sériques et remises en croissance soit en milieu sans sérum [transfection en absence de sérum], soit en milieu complet [transfection en présence de sérum] et 50 µl de mélange cytofectant [0,5 µg ADN/puits] sont ajoutés aux cellules [3 puits/condition lipide-DNA].

Quand les cellules sont transfectées en « absence de sérum » le milieu de croissance est supplémenté par la quantité appropriée de sérum 2 heures après la transfection.

L'efficacité de transfection est évaluée par une mesure de l'expression de la luciférase selon les recommandations données pour l'utilisation du kit Promega [Luciferase Assay System]. La toxicité des mélanges cytofectants est estimée par une mesure des concentrations protéiques dans les lysats cellulaires.

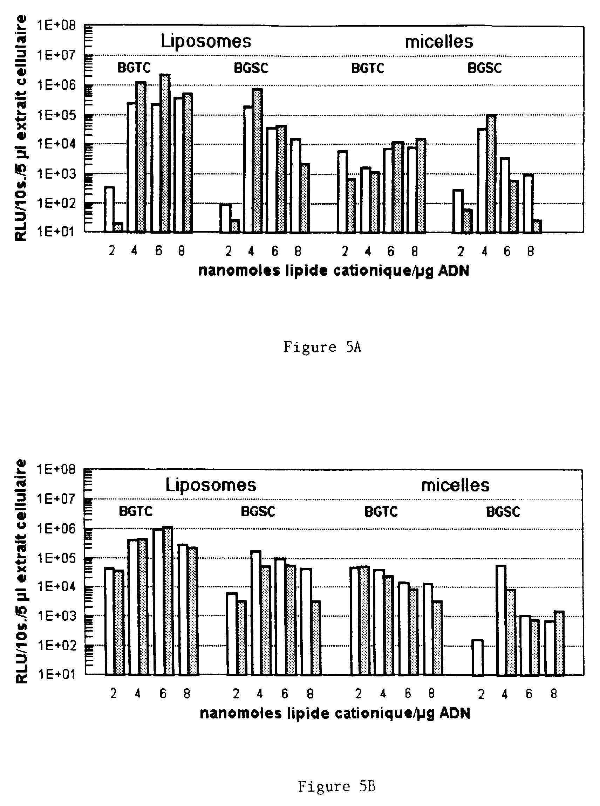

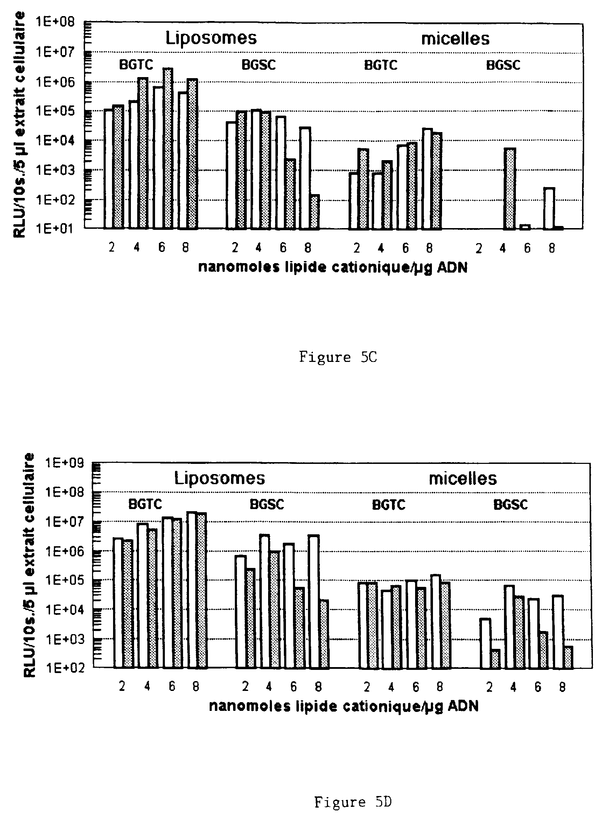

### 7.5 - Résultats (Figure 5)

Les résultats obtenus avec quatre types cellulaires [NIH3T3 - 293 - HepG2 - HeLa] montrent que, dans les conditions testées, les formulations sous forme de « liposomes » sont plus efficaces que celles sous forme de « micelles ». Par ailleurs, contrairement à ce qui est observé avec la plupart des préparations cytofectantes contenant des lipides cationiques, aucun effet inhibiteur significatif lié à la présence de sérum pour le BGTC sous forme de « liposomes » ou de « micelles » n'a pu être mis en évidence dans nos conditions de transfection.

### EXEMPLE 8

### EXPRESSION IN VIVO DE TRANSGENE DANS LES VOIES RESPIRATOIRES DE SOURIS AVEC DES LIPOSOMES BGTC/DOPE.

Pour ce faire, nous avons utilisé les liposomes cationiques BGTC/DOPE. L'obtention des agrégats ADN/liposomes et le protocole d'administration sont effectués comme décrits en matériel et méthode.

Les cellules X-gal sont détectées dans l'épithelium des voies respiratoires des souris traitées 48h après transfection au niveau des cellules du plasmide LacZ à l'aide des liposomes BGTC/DOPE. Les résultats sont présentés en figure 6.

La plupart des cellules transfectées sont localisées dans la trachée et seules quelques cellules X-gal sont observées dans les voies respiratoires secondaires. Ce type de distribution a déjà été reporté dans d'autres transfections cellulaires.

Nous avons en outre effectué une imunohistochimie pour identifier le produit du gène reporter. Majoritairement, ce sont les cellules matures qui sont transfectées au niveau de la surface de l'épithélium (figure 6A). Ces données montrent que les liposomes cationiques sont capables d'induire la transfection de gènes dans des cellules non proliferatives et totalement différenciées de l'épithélium des voies respiratoires. L'expression du transgène est également détectée dans les glandes sous-mucosales (figure 6B). La détection par marquage immunologique des cellules transfectées, en utilisant un anticorps monoclonal dirigé contre la β-galactosidase d'E coli, confirme cette expression dans les glandes sous-mucosales et l'épithélium de surface (figure 6C). L'expression transgénique est également mise en évidence dans les glandes sous-mucosales par coloration à l'immunoperoxidase avec un anticorps dirigé contre la protéine luciferase après transfection de p RSV-Luc avec BGTC/DOP (figure 6D).

Des contrôles négatifs sont effectués sans anticorps (figure 6E).

Ces résultats sont d'un intérêt tout particulier pour la thérapie génique dirigée contre la muscovidose. En effet, pour traiter cette pathologie, il est nécessaire de cibler les glandes sous-mucosales qui sont le site principal de l'expression de la CFTR dans les bronches humaines.

### EXEMPLE 9

### DOSAGE QUANTITATIF DE L'EFFICACITE DE TRANSFECTION DE BGTC/DOPE IN VIVO

Pour ce faire, les poumons et la trachée de souris, traitées avec du liposome BGTC/DOPE et du plasmide pRSV-Luc dans les conditions décrites en exemple 8, sont prélevées 48h après la transfection et l'activité luciférase dosée selon le protocole décrit dans matériel et méthode.
Les résultats sont présentés dans le tableau 3 ci-après.

**Tableau 3**

| VECTEURS | ACTIVITE LUCIFERASE (RLU/mg protéine) |
|---|---|
| BGTC/DOPE Liposomes : | |
| pRSV-Luc | 2.8 x 10⁵ (moyenne : 3 x 10⁴ - 8 x 10⁵) |
| | |
| pRSV-Lac Z | 0 |

| ADN nu | |
|---|---|
| | |
| pRSV-Luc | 0 |

L'activité luciférase est détectée de manière systématique dans les homogénats de trachée mais pas dans ceux des poumons. Cette observation est en accord avec les résultats précédents obtenus lors de l'examen de la distribution des cellules positives à l'Xgal. On note que l'expression luciferase est directement liée au plasmide d'expression puisque qu'aucune activité n'a pu être détectée dans le témoin contenant un plasmide LacZ. A titre témoin, il a été également été réalisé une transfection du plasmide sans vecteur. On n'observe dans cet essai témoin aucune expression de luciférase.

Ces données démontrent l'efficacité des liposomes BGTC/DOPE pour la transfection génique in vivo des voies respiratoires.

### EXEMPLE 10

### TRANSFERT DE GENE IN VIVO PAR INJECTION INTRAVEINEUSE OU INTRAPERITONEALE

Les formulations des lipides cationiques et l'ADN sont décrits dans l'exemple 7.

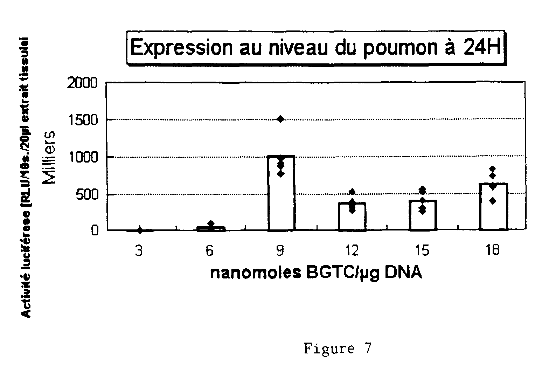

### 10.1 - Effet Dose des lipides cationiques (figures 7-8)

Les souris BalbC âgées de 5 semaines sont injectées par voie intraveineuse (veine queue) ou intrapéritonéale par 200 µl de mélange transfectant en solution dans du NaCl 37,5 mM, glucose 5% et l'expression de la luciférase est recherchée à 24 heures post-transfection dans le poumon après injection intraveineuse, et dans le foie et la rate après injection intrapéritonéale. Les organes sont prélevés à froid dans du tampon de lyse [Promega E153A] additionné d'inhibiteurs de protéase [Boehringer 1697498] et homogénéisés avec un broyeur Heidolph DIAX600. L'activité luciférase est recherchée dans le surnageant 14000g des extraits tissulaires.

### Injection intraveineuse

Chaque souris reçoit 50 µg d'ADN complexé par différentes concentrations de BGTC sous forme de « liposomes ». Le rapport optimum en nanomoles lipide cationique/µg ADN observé est de 9 (figure 7); aucune toxicité n'a pu être mise en évidence pour des doses allant jusqu'à 900 nanomoles de BGTC injectées.

### Injection intrapéritonéale :

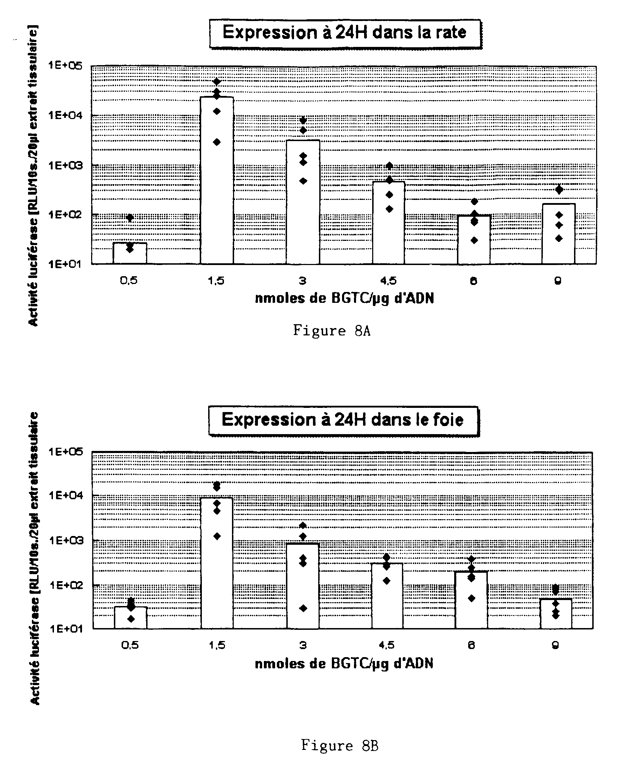

Le BGTC sous forme de « liposomes » a été injecté avec 100 µg d'ADN par souris. Aussi bien dans le foie (figure 8B) que dans la rate (figure 8A), le maximum d'expression est obtenu pour 1,5 nanomoles BGTC/µg d'ADN.

### 10.2 - Cinétique d'expression après injection intraveineuse

Nous avons recherché la biodistribution de l'expression de la luciférase dans 7 organes en fonction du temps suivant l'injection de 200 µl de mélange « liposomes »BGTC/ADN dans du NaCl 37,5 mM, glucose 5% [ 50 µg ADN et 9 nanomoles BGTC/µg ADN] dans la veine de la queue de souris BalbC âgées de 5 semaines. Les résultats sont exprimés en picogramme de luciférase extraite par organe suivant une courbe de calibration avec de la luciférase commercialisée sous forme cristallisée. La limite de détection se situe entre 0,5 et 1 picogramme de luciférase.

Nous mettons en évidence que, dans les conditions testées, le maximum d'expression est obtenu à 23 heures post-transfection pour 6 des 7 organes étudiés, à savoir le diaphragme, le gastrocnémien, le coeur, le poumon, le rein et la rate. L'expression au niveau du foie semble plus précoce. Le maximum d'expression est obtenu au niveau pulmonaire avec un taux de 0,5 nanogramme à 23 heures post-transfection.

## Revendications

1. Composé de formule générale I et ses sels 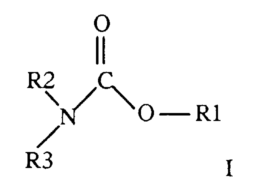 dans laquelle:
- R1 représente un dérivé du cholestérol,
R2 et R3 représentent indépendamment l'un de l'autre un atome d'hydrogène ou un groupement de formule générale (II) avec au moins l'un d'entre eux différent d'un atome d'hydrogène, 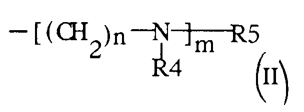 dans laquelle:
- n et m représentent indépendamment l'un de l'autre et de manière distincte entre les groupements R2 et R3 un entier compris entre 0 et 4
- R4 et R5 représentent indépendamment l'un de l'autre un atome d'hydrogène ou un groupement de formule (III) avec au moins l'un d'entre eux différent d'un atome d'hydrogène : 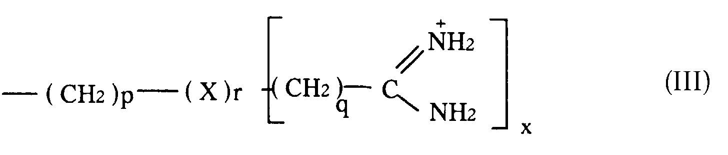
dans laquelle
p et q représentent indépendamment l'un de l'autre un entier compris entre 0 et 4 et r est égal à 0 ou 1, avec pour r égal à 1
X représentant un groupement NH et x étant alors égal à 1 ou
X représentant un atome d'azote et x étant alors égal à 2.
avec p, q et r pouvant varier indépendamment entre les groupements R4 et R5.

2. Composé selon la revendication 1 caractérisé en ce que R2 et R3 y représentent indépendamment l'un de l'autre un atome d'hydrogène ou un groupement de formule générale IV 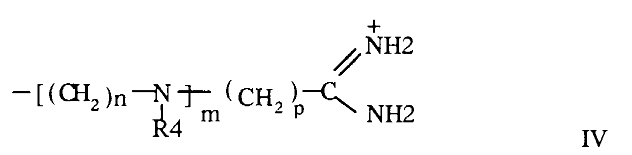 dans laquelle
n, m et p sont définis comme précédemment et R4 représente un atome d'hydrogène ou un groupement de formule V 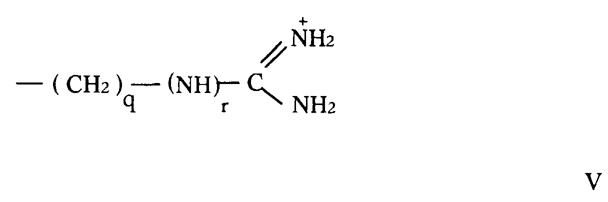
avec q et r définis comme précédemment,
et avec m, n, p q et r pouvant varier de manière indépendante entre les différents groupements R2 et R3.

3. Composé selon la revendication 1 ou 2 caractérisé en ce qu'il s'agit d'un composé représenté par l'une des sous formules générales suivantes:
(H₂N)₂⁺C-NH-(CH₂)₂-NH-COO-R1 VI
[(H₂N)₂⁺C-NH-(CH₂)₃][(H₂N)₂⁺C-NH-(CH₂)₄]-N-COO-R1 VII
[(H₂N)₂⁺C-NH-(CH₂)₂]₂-N-(CH₂)₂-NH-COO-R1 VIII
[(H₂N)₂⁺C-(CH₂)₂]₂N-COO-R1 IX
ou
[[(H₂N)₂⁺C-(CH₂)₂]₂-N-(CH₂)₂]₂N-COO-R1 X
dans lesquelles R1est défini selon la revendication 1.

4. Composé selon l'une des revendications précédentes caractérisé en qu'il est choisi parmi
[[(H₂N)₂⁺C-(CH₂)₂]₂-N-(CH₂)₂]₂N-COO-R1
[(H₂N)₂⁺C-NH-(CH₂)₃][(H₂N)₂⁺C-NH-(CH₂)₄]-N-COO-R1
et
[(H₂N)₂⁺C-(CH₂)₂]₂N-COO-R1
dans lesquels R1 représente un groupement cholesteryle.

5. Composé selon l'une des revendications précédentes caractérisé en ce qu'il s'agit du 3β-O-[N-(3-guanidinopropyl), N-(4-guanidinobutyl)]carbamoylcholestérol ("BGSC").

6. Composé selon l'une des revendications précédentes caractérisé en ce qu'il s'agit du 3β-O-[N-(N',N'-bis-(2-guanidinoéthyl)-2-aminoéthyl)carbamoyl]cholestérol ("BGTC").

7. Composition pharmaceutique caractérisée en ce qu'elle comprend au moins un composé selon l'une des revendications précédentes.

8. Composition pharmaceutique selon la revendication 7 caractérisée en ce que le composé est le 3β-O-[N-(3-guanidinopropyl); N-(4-guanidinobutyl)]carbamoylcholestérol ("BGSC").

9. Composition pharmaceutique selon la revendication 7 caractérisée en ce que le composé est le 3β-O-[N-(N',N'-bis-(2-guanidinoéthyl) 2-aminoéthyl)carbamoyl]cholestérol ("BGTC").

10. Composition pharmaceutique selon l'une des revendications 7 à 9 caractérisée en ce qu'elle contient en outre un acide nucléique.

11. Composition selon la revendication 10 caractérisée en ce que l'acide nucléique est un acide désoxyribonucléique.

12. Composition selon la revendication 10 caractérisée en ce que l'acide nucléique est un acide ribonucléique.

13. Composition selon l'une des revendications 10 à 12 caractérisée en ce que l'acide nucléique comporte un gène thérapeutique.

14. Composition pharmaceutique comprenant un composé selon la revendication 1, un lipide neutre et un acide nucléique.

15. Composition pharmaceutique selon la revendication 14 caractérisée en ce que le composé est choisi parmi le BGTC et le BGSC.

16. Composition pharmaceutique selon la revendication 14 caractérisé en ce que le lipide neutre est la 1,2-dioléoyl-sn-glycéro-3-phosphoéthanolamine ("DOPE").

17. Composition pharmaceutique selon la revendication 14 caractérisée en ce que l'acide nucléique est un acide désoxyribonucléique.

18. Composition pharmaceutique selon la revendication 14 caractérisée en ce que l'acide nucléique est un acide ribonucléique.

19. Composition pharmaceutique selon l'une des revendications 17 ou 18 caractérisée en ce que l'acide nucléique comporte un gène thérapeutique.

20. Composition pharmaceutique selon l'une quelconque des revendications 7 à 19 caractérisée en ce qu'elle comprend un véhicule pharmaceutiquement acceptable pour une formulation injectable.

21. Composition pharmaceutique selon l'une quelconque des revendications 7 à 19 caractérisée en ce qu'elle comprend un véhicule pharmaceutiquement acceptable pour une application sur la peau et/ou les muqueuses.

22. Utilisation d'un composé selon l'une des revendications de 1 à 6 pour préparer des compositions pharmaceutiques destinées à la transfection in vitro, ex vivo et/ou in vivo de cellules.

## Patentansprüche

1. Verbindung der allgemeinen Formel I und Salze davon 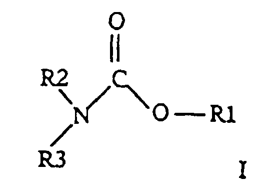 worin
- R1 ein Cholesterinderivat bezeichnet,
- R2 und R3 unabhängig voneinander ein Wasserstoffatom oder eine Gruppierung der allgemeinen Formel (II) bezeichnen, wobei mindestens einer der Reste sich von einem Wasserstoffatom unterscheidet, 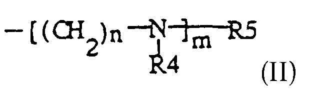
worin
- n und m unabhängig voneinander und unterschiedlich zwischen den Gruppierungen R2 und R3 eine ganze Zahl zwischen 0 und 4 bezeichnen,
- R4 und R5 unabhängig voneinander ein Wasserstoffatom oder eine Gruppierung der Formel (III) bezeichnen, wobei mindestens einer der Reste sich von einem Wasserstoffatom unterscheidet: 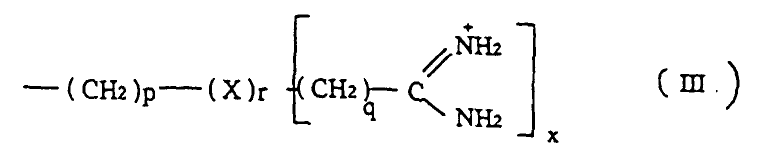
worin
p und q unabhängig voneinander eine ganze Zahl zwischen 0 und 4 bezeichnen und r den Wert 0 oder 1 hat, wobei für r gleich 1
X eine NH-Gruppe bezeichnet und x nun gleich 1 ist oder
X ein Stickstoffatom bezeichnet und x nun gleich 2 ist,
wobei p, q und r unabhängig voneinander zwischen den Gruppierungen R4 und R5 variieren können.

2. Verbindung nach Anspruch 1, dadurch **gekennzeichnet**, dass R2 und R3 unabhängig voneinander ein Wasserstoffatom oder eine Gruppierung der allgemeinen Formel IV 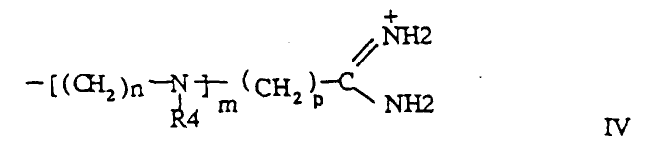 bezeichnen,
worin
n, m und p wie vorstehend definiert sind und R4 ein Wasserstoffatom oder eine Gruppierung der Formel V 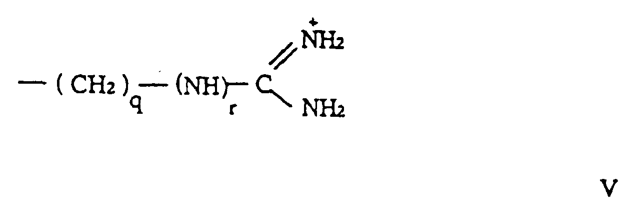 bezeichnet,
wobei q und r wie vorstehend definiert sind
und wobei m, n, p, q und r unabhängig voneinander zwischen den verschiedenen Gruppierungen R2 und R3 variieren können.

3. Verbindung nach Anspruch 1 oder 2, dadurch **gekennzeichnet**, dass es sich um eine Verbindung, wiedergegeben durch eine der nachstehenden allgemeinen Formeln, handelt:
(H₂N)₂⁺C-NH-(CH₂)₂-NH-COO-R1 VI
[(H₂N)₂⁺C-NH-(CH₂)₃][(H₂N)₂⁺C-NH-(CH₂)₄]-N-COO-R1 VII
[(H₂N)₂⁺C-NH-(CH₂)₂]₂-N-(CH₂)₂-NH-COO-R1 VIII
[(H₂N)₂⁺C-(CH₂)₂]₂N-COO-R1 IX
oder
[[(H₂N)₂⁺C-(CH)₂]₂]₂-N-(CH₂)₂]₂N-COO-R1 X
worin R1 wie in Anspruch 1 definiert ist.

4. Verbindung nach einem der vorstehenden Ansprüche, dadurch **gekennzeichnet**, dass sie ausgewählt ist aus
[(H₂N)₂⁺C-(CH₂)₂]₂-N-(CH₂)₂] N-COO-R1
[(H₂N)₂⁺C-NH-(CH₂)₃][(H₂N)₂⁺C-NH-(CH₂)₄]-N-COO-R1
und
[(H₂N)₂⁺C-(CH₂)₂]₂N-COO-R1
worin R1 eine Cholesterylgruppierung bezeichnet.

5. Verfahren nach einem der vorstehenden Ansprüche, dadurch **gekennzeichnet**, dass es sich um 3β-O-[N-(3-Guanidinopropyl)-N-(4-guanidinobutyl)]carbamoylcholesterin (BGSC) handelt.

6. Verbindung nach einem der vorstehenden Ansprüche, dadurch **gekennzeichnet**, dass es sich um 3β-O-[N-(N',N'-Bis-(2-guanidinoethyl)-2-aminoethyl)carbamoyl]cholesterin (BGTC) handelt.

7. Pharmazeutische Zusammensetzung, dadurch **gekennzeichnet**, dass sie mindestens eine Verbindung nach einem der vorstehenden Ansprüche umfasst.

8. Pharmazeutische Zusammensetzung nach Anspurch 7, dadurch **gekennzeichnet**, dass die Verbindung 3β-O-[N-(3-Guanidinopropyl)-N-(4-guanidinobutyl)]carbamoylcholesterin (BGSC) ist.

9. Pharmazeutische Zusammensetzung nach Anspruch 7, dadurch **gekennzeichnet**, dass die Verbindung 3β-O-[N-(N',N'-Bis-(2-guanidinoethyl)-2-aminoethyl)carbamoyl]cholesterin (BGTC) ist.

10. Pharmazeutische Zusammensetzung nach einem der Ansprüche 7 bis 9, dadurch **gekennzeichnet**, dass sie weiterhin eine Nukleinsäure enthält.

11. Zusammensetzung nach Anspruch 10, dadurch **gekennzeichnet**, dass die Nukleinsäure Desoxyribonukleinsäure ist.

12. Zusammensetzung nach Anspruch 10, dadurch **gekennzeichnet**, dass die Nukleinsäure Ribonukleinsäure ist.

13. Zusammensetzung nach einem der Ansprüche 10 bis 12, dadurch **gekennzeichnet**, dass die Nukleinsäure ein therapeutisches Gen enthält.

14. Pharmazeutische Zusammensetzung, umfassend eine Verbindung nach Anspruch 1, ein Neutrallipid und eine Nukleinsäure.

15. Pharmazeutische Zusammensetzung nach Anspruch 14, dadurch **gekennzeichnet**, dass die Verbindung aus BGTC und BGSC ausgewählt ist.

16. Pharmazeutische Zusammensetzung nach Anspruch 14, dadurch **gekennzeichnet**, dass das Neutrallipid 1,2-Dioleoyl-sn-glycero-3-phosphoethanolamin (DOPE) ist.

17. Pharmazeutische Zusammensetzung nach Anspruch 14, dadurch **gekennzeichnet**, dass die Nukleinsäure eine Desoxyribonukleinsäure ist.

18. Pharmazeutische Zusammensetzung nach Anspruch 14, dadurch **gekennzeichnet**, dass die Nukleinsäure eine Ribonukleinsäure ist.

19. Pharmazeutische Zusammensetzung nach einem der Ansprüche 17 oder 18, dadurch **gekennzeichnet**, dass die Nukleinsäure ein therapeutisches Gen enthält.

20. Pharmazeutische Zusammensetzung nach einem der Ansprüche 7 bis 19, dadurch **gekennzeichnet**, dass sie einen pharmazeutisch verträglichen Träger für eine injizierbare Formulierung umfasst.

21. Pharmazeutische Zusammensetzung nach einem der Ansprüche 7 bis 19, dadurch **gekennzeichnet**, dass sie einen pharmazeutisch verträglichen Träger für eine Anwendung auf der Haut und/oder den Schleimhäuten umfasst.

22. Verwendung einer Verbindung nach einem der Ansprüche 1 bis 6 zur Herstellung von pharmazeutischen Zusammensetzungen zur in-vitro-, ex-vivo- und/oder in-vivo-Transfektion von Zellen.

## Claims

1. Compound of general formula I and its salts 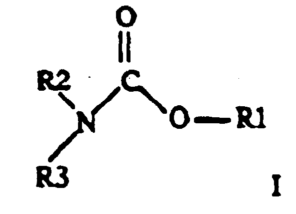 in which:
- R1 represents a cholesterol derivative,
R2 and R3 represent, independently of each other, a hydrogen atom or a group of general formula (II) with at least one of them being different from a hydrogen atom, 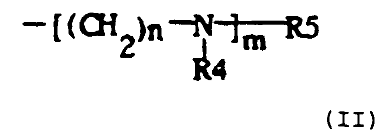 in which:
- n and m represent, independently of each other and distinctly between the groups R2 and R3, an integer between 0 and 4,
- R4 and R5 represent, independently of each other, a hydrogen atom or a group of formula (III) with at least one of them being different from a hydrogen atom: 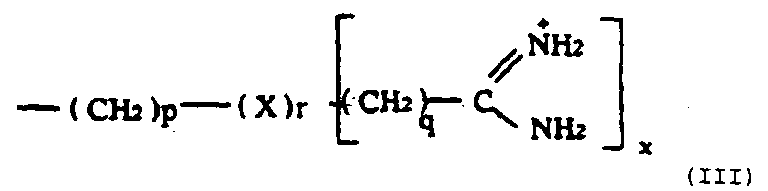
in which
p and q represent, independently of each other, an integer between 0 and 4 and r is equal to 0 or 1, with r equal to 1
X representing an NH group and x then being equal to 1 or
X representing a nitrogen atom and x then being equal to 2.
it being possible for p, q and r to vary independently between the groups R4 and R5.

2. Compound according to claim 1, characterized in that R2 and R3 represent therein, independently of each other, a hydrogen atom or a group of general formula IV 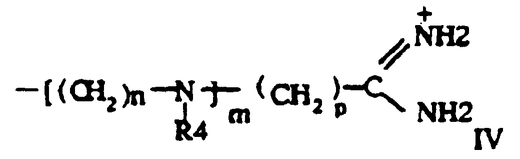 in which
n, m and p are as defined above and R4 represents a hydrogen atom or a group of formula V 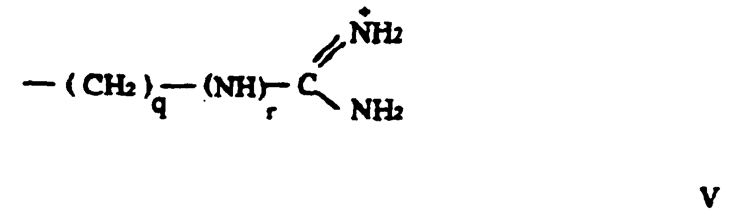 with q and r as defined above,
and it being possible for m, n, p q and r to vary independently between the different groups R2 and R3.

3. Compound according to claim 1 or 2, characterized in that it is a compound represented by one of the following general subformulae:
(H₂N)₂⁺C-NH-(CH₂)₂-NH-COO-R1 VI
[(H₂N)₂⁺C-NH-(CH₂)₃][(H₂N)₂C⁺-NH-(CH₂)₄]-N-COO-R1 VII
[(H₂N)₂⁺C-NH-(CH₂)₂]₂-N-(CH₂)₂-NH-COO-R1 VIII
[(H₂N)₂⁺C-(CH₂)₂]₂N-COO-R1 IX
[[(H₂N)₂⁺C-(CH)₂]₂]₂-N-(CH₂)₂]₂N-COO-R1 X
in which R1 is defined according to claim 1.

4. Compound according to one of the preceding claims, characterized in that it is chosen from
[(H₂N)₂⁺C-(CH₂)₂]₂-N-(CH₂)₂]₂N-COO-R1
[(H₂N)₂⁺C-NH-(CH₂)₃][(H₂N)₂⁺C-NH-(CH₂)₄]-N-COO-R1
and
[(H₂N)₂⁺C-(CH₂)₂]₂N-COO-R1
in which R1 represents a cholesteryl group.

5. Compound according to one of the preceding claims, characterized in that it is 3β-O-[N-(3-guanidinopropyl)-N-(4-guanidinobutyl)]carbamoylcholesterol ("BGSC").

6. Compound according to one of the preceding claims, characterized in that it is 3β-O-[N-(N',N'-bis-(2-guanidinoethyl)-2-aminoethyl)-carbamoyl]cholesterol ("BGTC").

7. Pharmaceutical composition, characterized in that it comprises at least one compound according to one of the preceding claims.

8. Pharmaceutical composition according to claim 7, characterized in that the compound is 3β-O-[N-(3-guanidinopropyl)-N-(4-guanidinobutyl)]carbamoylcholesterol ("BGSC").

9. Pharmaceutical composition according to claim 7, characterized in that the compound is 3β-O-[N-(N',N'-bis-(2-guanidinoethyl)-2-aminoethyl)carbamoyl]cholesterol ("BGTC").

10. Pharmaceutical composition according to one of claims 7 to 9, characterized in that it contains, in addition, a nucleic acid.

11. Composition according to claim 10, characterized in that the nucleic acid is a deoxyribonucleic acid.

12. Composition according to claim 10, characterized in that the nucleic acid is a ribonucleic acid.

13. Composition according to one of claims 10 to 12, characterized in that the nucleic acid comprises a therapeutic gene.

14. Pharmaceutical composition comprising a compound according to claim 1, a neutral lipid and a nucleic acid.

15. Pharmaceutical composition according to claim 14, characterized in that the compound is chosen from BGTC and BGSC.

16. Pharmaceutical composition according to claim 14, characterized in that the neutral lipid is 1,2-dioleoyl-sn-glycero-3-phosphoethanolamine ("DOPE").

17. Pharmaceutical composition according to claim 14, characterized in that the nucleic acid is a deoxyribonucleic acid.

18. Pharmaceutical composition according to claim 14, characterized in that the nucleic acid is a ribonucleic acid.

19. Pharmaceutical composition according to either of claims 17 and 18, characterized in that the nucleic acid comprises a therapeutic gene.

20. Pharmaceutical composition according to any one of claims 7 to 19, characterized in that it comprises a pharmaceutically acceptable vehicle for an injectable formulation.

21. Pharmaceutical composition according to any one of claims 7 to 19, characterized in that it comprises a pharmaceutically acceptable vehicle for application to the skin and/or the mucous membranes.

22. Use of a compound according to one of claims 1 to 6, for preparing pharmaceutical compositions intended for the in vitro, ex vivo and/or in vivo transfection of cells.
